# FASCICULE DE BREVET EUROPEEN

(11) **EP 0 986 390 B1**
(45) Date de publication et mention de la délivrance du brevet: **25.09.2002**
(21) Numéro de dépôt: 98929515.9
(22) Date de dépôt: 08.06.1998
(51) Int. Cl.: A61K 31/49, A61P 43/00, A61P 9/06, A61P 21/00, A61P 33/06

(54) **COMPOSITIONS PHARMACEUTIQUES CONTENANT DU DICHLORHYDRATE DE CINCHONINE**
PHARMAZEUTISCHE ZUSAMMENSETZUNGEN VON CINCHONIN DIHYDROCHLORID
PHARMACEUTICAL COMPOSITIONS CONTAINING CINCHONINE DICHLORHYDRATE

(30) Priorité: 11.06.1997 FR 9707234
(43) Date de publication de la demande: 22.03.2000
(73) Titulaire: DEBIOPHARM S.A., 1000 Lausanne 9 (CH)
(72) Inventeur: GENNE, Philippe, F-21240 Ralant (FR); IBRAHIM, Houssam, CH-1255 Veyrier (CH)
(74) Mandataire: Nargolwalla, Cyra
(86) Numéro de dépôt international: FR9801166
(87) Numéro de publication internationale: WO98056383

(56) Documents cités:
- WO-A-92/14467
- T.J. JANJIC ET AL.: "Two-phase buffer systems with diprotic acids" ANAL. CHIM. ACTA, vol. 152, 1983, pages 229-237, XP002056486

## Description

La présente invention a pour objet des compositions pharmaceutiques contenant du dichlorhydrate de cinchonine comme principe actif. Plus précisément, les compositions pharmaceutiques selon l'invention contiennent du dichlorhydrate de cinchonine ayant une pureté d'au moins 95 %.

L'invention concerne également un procédé pour la préparation du dichlorhydrate de cinchonine.

Les compositions pharmaceutiques selon l'invention peuvent être utilisées en tant que substances inhibitrices du phénomène dit de la "résistance multidrogue" (RMD, en anglais, "multiple drug resistance"). Plus particulièrement, les compositions pharmaceutiques selon l'invention peuvent être utilisées pour les traitements de certaines maladies cancéreuses qui manifestent ce phénomène de la RMD.

Les compositions pharmaceutiques selon l'invention peuvent également être utilisées pour le traitement de l'arythmie, en tant que substances antispasmodiques, ainsi que dans le traitement du paludisme, en cas de résistance aux médicaments classiques connus et en association avec ces médicaments.

Le phénomène de la RMD est connu notamment en ce qui concerne les cancers, mais se manifeste également dans le cas d'autres maladies telles que le paludisme. Il existe d'une part des cancers ayant une RMD innée et, d'autre part, des cancers qui développent une RMD suite aux traitements à base d'agents anticancéreux, tels que notamment les anthracyclines. Le phénomène de la RMD dans les cancers a pour effet d'inhiber la cytotoxicité de certains agents anticancéreux voire même de les rendre inefficaces.

La cinchonine est une substance naturelle, extraite des écorces de quinquina et qui appartient à la famille des alcaloïdes. La cinchonine a une activité inhibitrice vis-à-vis du phénomène de RMD. Son utilisation pour la préparation de compositions pharmaceutiques destinées au traitement de tumeurs cancéreuses qui développent le phénomène de RMD est décrite dans le brevet européen EP n° 0052608 au nom de la Société Demanderesse DEBIOPHARM S.A.

Les compositions pharmaceutiques contenant de la cinchonine et destinées au traitement du phénomène de RMD présentent, cependant, un certain nombre d'inconvénients.

Ces inconvénients proviennent principalement du fait que la cinchonine est quasiment insoluble dans l'eau. Ceci rend difficile son administration aux patients sous forme de solution buvable ou parentérale. De plus, la faible solubilité dans l'eau de la cinchonine rend ses propriétés de biodisponibilité non satisfaisantes pour une administration orale. La cinchonine étant une substance toxique, ayant des effets secondaires indésirables, il serait souhaitable de pouvoir améliorer sa biodisponibilité, ce qui permettrait de réduire les doses administrées oralement et, ainsi, les effets secondaires.

La Société Demanderesse a eu le mérite de trouver qu'un sel de la cinchonine, le dichlorhydrate de cinchonine, non seulement répondait aux critères de solubilité et de biodisponibilité souhaités pour une formulation ganélique tout en conservant les propriétés thérapeutiques recherchées, mais qu'en outre il présentait une très bonne stabilité en solution, favorisant de ce fait la formulation de préparations acqueuses notamment sous forme injectable.

Par ailleurs, la Société Demanderesse a également constaté que la biodisponibilité du dichloryhdrate de cinchonine était plus constante d'un patient à l'autre. Autrement dit, la variabilité de l'effet thérapeutique est moins grande quand on traite les patients avec le dichlorhydrate de cinchonine qu'avec la cinchonine ou le monochlorhydrate de cinchonine.

La composition pharmaceutique selon l'invention contient, comme principe actif, du dichlorhydrate de cinchonine. Plus particulièrement, la composition pharmaceutique selon l'invention contient du dichlorhydrate de cinchonine présentant une pureté au moins égale à 95 %. De préférence, le dichlorhydrate de cinchonine dans la composition pharmaceutique selon l'invention présente une pureté au moins égale à 99 % et plus préférentiellement encore au moins égale à 99,9 %.

Selon un mode de réalisation préférentiel, la composition pharmaceutique, objet de l'invention, contient du dichlorhydrate de cinchonine en combinaison avec au moins un autre principe actif compatible avec le dichlorhydrate de cinchonine et éventuellement au moins un autre composé pharmaceutiquement acceptable.

Les autres principes actifs peuvent avantageusement être choisis dans le groupe comprenant les agents anti-cancéreux, antipaludiens et inhibiteurs de la résistance multidrogue.

En tant qu'agents anti-cancéreux, on peut citer de manière non exhaustive la Doxorubicine, la Vinorelbine, l'Etoposide, le Taxol, la Puromicine et, de manière générale, tous les agents cytotoxiques impliqués dans la résistance multiple aux drogues.

En tant qu'agents antipaludiens, on peut citer de manière non exhaustive la quinine, la chloroquinine et tout autre agent qui provoque un phénomène de résistance.

En tant qu'inhibiteurs de la RMD, on peut citer de manière non exhaustive une ou plusieurs substances connues choisies parmi l'amiodarone, la quinine, la quinidine, la cinchonidine, le vérapamil, la cyclosporine A, les céphalosporines, le bipéridène, la lidocaïne, la chlorpromazine, la pentazocine, la prométhazine, le potassium canmrénoate, l'amitriptyline, le propanolol, le déméthoxyvérapamil, le diltiazème, la thioridazine, la trifluopérazine, la chloroquine, la sdb-éthylène diamine, la réserpine, le tamoxifène, le torémifène, l'hydrocortisone, la progestérone, le salbutamol et leurs dérivés acyles ou esters.

Comme autre composé pharmaceutiquement acceptable utilisé dans les compositions de l'invention, on peut avantageusement utiliser des substances cytotoxiques sensibles au phénomène de la RMD, qui augmenteront l'action du dichlorhydrate de cinchonine. De telles substances sont mentionnées ci-après ; cette énumération n'est pas exhaustive pour autant.

Ce sont pour l'essentiel des substances hydrophobes, ayant en commun un résidu azoté chargé positivement, tels les alcaloïdes de la vinca, les anthracyclines ou produits analogues, les épipodophyllotoxines ou les antibiotiques antitumoraux par exemple. On peut citer en particulier la vincristine, la vinblastine, la vindésine, la vinorelbine, la doxorubicine, la déoxydoxorubicine, la tétrahydropyranyladriamycine, l'épidoxorubicine, l'aclacinomycine, la déméthoxydaunorubicine, la daunorubicine, le m-amsa, la mitoxantrone, le bisanthrène, le déméthoxydaunorubisanthrène, la mithramycine, l'actinomycine D, la puromycine, l'étoposide, le ténoposide, l'émétine, l'éthidium bromide, la cytochalasine, la colchicine et le taxol.

D'autres composés pharmaceutiquement acceptables peuvent bien entendu être incorporés dans la composition selon l'invention, tels que des charges, des colorants, des excipients, des édulcorants, etc.

Les doses et les fréquences d'administration optimales de la composition selon l'invention dépendront du type de cancer ou autre maladie traité, de la nature des autres principes actifs éventuellement utilisés en combinaison avec le dichlorhydrate de cinchonine ainsi que du patient traité ou encore d'autres facteurs que l'homme du métier sera en mesure d'appréhender.

L'invention permet donc de proposer un traitement thérapeutique approprié des maladies présentant à des degrés divers une résistance aux médicaments classiquement utilisés pour les traiter. En particulier, grâce à l'invention, on est en mesure de proposer un traitement thérapeutique approprié de nombreuses maladies cancéreuses présentant à des degrés divers une résistance aux agents anti-cancéreux. A ce propos, on peut citer entre autres la leucémie aigüe myéloblastique, la leucémie aigüe lymphoblastique, le neuroblastome, le cancer du poumon à petites cellules, le cancer de l'ovaire, le lymphome malin non hodgkinien et le plasmocytome diffus. Ce sont des cancers qui peuvent présenter une RMD induite, en réponse aux traitements avec un agent cytotoxique.

On peut également traiter des cancers présentant une RMD innée. Ce sont, par exemple, l'adénocarcinome du côlon, l'adénocarcinome du rein, le carcinome corticosurrénalien, le phéochromocytome, les sarcomes de l'enfant et la leucémie secondaire. Cette liste n'est cependant pas exhaustive.

Le dichlorhydrate de cinchonine présente une très bonne stabilité en solution aqueuse, ce qui le rend tout à fait approprié pour la préparation de solutions injectables. Ainsi, le dichlorhydrate de cinchonine peut être administré aisément localement au niveau des zones à traiter, limitant ainsi les éventuels effets secondaires sur les parties saines.

La Société Demanderesse a également mis au point un procédé permettant d'obtenir du dichlorhydrate de cinchonine présentant une pureté au moins égale à 95 %.

Selon le procédé objet de l'invention, on prépare le dichlorhydrate de cinchonine en ajoutant de l'acide chlorhydrique à la cinchonine ou au monochlorhydrate de cinchonine afin d'obtenir le dichlorhydrate de cinchonine, et on purifie la cinchonine, le monochlorhydrate de cinchonine et/ou le dichlorhydrate de cinchonine. Ces étapes peuvent être réalisées dans un ordre quelconque. Avantageusement, le procédé de préparation du dichlorhydrate de cinchonine selon l'invention comprend une étape finale de filtration stérilisante puis un séchage par lyophilisation du produit obtenu.

Les étapes de purification sont avantageusement réalisées par chromatographie liquide haute performance.

Selon un mode de réalisation préférentiel du procédé de préparation du dichlorhydrate de cinchonine à partir de la cinchonine, on ajoute l'acide chlorhydrique en deux étapes, la cinchonine étant dans une première étape transformée en monochlorhydrate de cinchonine, puis, dans une deuxième étape, le monochlorhydrate de cinchonine est transformé en dichlorhydrate de cinchonine. Les deux neutralisations auront lieu à température ambiante sous agitation constante. Dans la première étape, la cinchonine de base est mélangée avec de l'acide chlorhydrique en quantités équivalentes dans de l'eau. Si nécessaire, le mélange est chauffé jusqu'à ce que la réaction soit terminée. La solution est évaporée jusqu'à saturation du sel et on laisse cristalliser.

On ajoute ensuite le MCC obtenu à une solution d'acide chlorhydrique dilué sous agitation. On ajuste le pH à une valeur de 2.3 - 2.4 avec de l'acide chlorhydrique, on filtre puis on transfère la solution sur un plateau pour séchage.

Il peut aussi être envisagé de neutraliser, en une seule étape, la cinchonine en dichlorhydrate de cinchonine. La cinchonine et/ou le dichlorhydrate de cinchonine peuvent être purifiés par chromatographie liquide haute performance.

La neutralisation par HCl est réalisée selon les procédés bien connus par l'homme du métier. Ainsi, l'acide chlorhydrique peut être ajouté par petites fractions avec des étapes d'homogénéisation entre chaque étape.

L'avantage de procéder par plusieurs étapes réside dans le fait que l'on peut fixer des contrôles en cours de fabrication à l'issue de chaque étape permettant ainsi un meilleur suivi des impuretés et des rendements .

La composition pharmaceutique selon l'invention peut être utilisée pour le traitement du phénomène de la résistance multidrogue. Elle peut ainsi être utilisée pour le traitement des cancers, de l'arythmie ou en tant que produit antispasmodique. Elle peut également être utilisée en association avec des médicaments connus pour le traitement du paludisme en cas de résistance à ces médicaments.

L'invention sera mieux comprise à l'aide des exemples non-limitatifs qui suivent.

### EXEMPLE 1 :

### Préparation du dichlorhydrate de cinchonine à partir du monochlorhydrate de cinchonine

Une quantité de 7,074 g du monochlorhydrate de cinchonine, obtenue chez FLUKA, est ajoutée à 50 ml d'eau, sous agitation, à température ambiante. Le pH de cette solution est amené à 2,3 ± 0,1 après addition de 19,7 ml d'acide chlorhydrique (1N).

Cette solution est maintenue à la température ambiante sous agitation durant une heure jusqu'à stabilisation complète du pH. La solution est ensuite soumise à une filtration stérilisante par passage sur un filtre millipore 0,2 µm.

La solution est fractionnée puis congelée à -80°C au contact de l'azote liquide, puis lyophilisée pendant 70 heures. On obtient ainsi 5,86 g de poudre de dichlorhydrate de cinchonine lyophilisé.

### EXEMPLE 2 :

### Structure chimique et pureté du dichlorhydrate de cinchonine obtenu à l'Exemple 1.

Une fraction du produit obtenu à l'Exemple 1 ci-dessus est analysée par spectrométrie IR dans du bromure de potassium sur un spectromètre IR de type Perkin-Elmer, Model 881.

Le spectre obtenu est conforme au spectre du dichlorhydrate de cinchonine pur.

Une fraction du produit obtenu à l'exemple 1 est diluée dans de l'eau. L'échantillon obtenu est analysé par spectrométrie UV.

Le spectre obtenu est conforme au spectre du dichlorhydrate de cinchonine pur.

Le point de fusion du dichlorhydrate de cinchonine obtenu à l'Exemple 1 est déterminé à l'aide d'un banc de Kofler (Kofler Heizbank, Reichert, Allemagne). On a obtient une valeur de 232 ± 3°C.

### EXEMPLE 3 :

### Stabilité du dichlorhydrate de cinchonine obtenu à l'Exemple 1.

Des spectres 1H et 13C ont été accumulés respectivement sur des échantillons de 20 mg et 100mg dans 0.5 ml de méthanol deutérié.

Les spectres RMN sont réalisés à l'aide d'un spectromètre Bruker DRX 500 équipé d'une sonde dite "reverse".

La solution aqueuse est conservée à la température ambiante et à la lumière pendant 30 jours. Après 30 jours, des spectres RMN sont réalisés comme précédemment.

Les spectres RMN obtenus au jour 0 et au jour 30 sont inchangés.

Le dichlorhydrate de cinchonine présente donc une stabilité remarquable.

### EXEMPLE 4 :

### Biodisponibilité in vivo du dichlorhydrate de cinchonine.

Des essais comparatifs ont été effectués pour démontrer la meilleure biodisponibilité du dichlorhydrate de cinchonine *in vivo*.

Ces essais ont été effectués sur quatre chiens "Beagle", dont deux mâles et deux femelles.

Chaque chien a reçu, au jour 1, un dosage de 50 mg par kg du dichlorhydrate de cinchonine, et au jour 8, un dosage équivalent de monochlorhydrate de cinchonine. Des échantillons de sang ont été prélevés à des intervalles réguliers suivant chacune de ces administrations.

Les résultats d'une étude pharmacocinétique sont résumés dans le tableau ci-dessous :

**Tableau**

| **ANIMAL** | **M 1** | | **M 2** | | **F 1** | | **F 2** | |
|---|---|---|---|---|---|---|---|---|
| **Sel de Cinchonine** | DCC | MCC | DCC | MCC | DCC | MCC | DCC | MCC |
| **Dose mg/kg** | 50 | 50 | 50 | 50 | 50 | 50 | 50 | 50 |
| **C**_{**20**}**min.** | 0.23 | 0 | 2.1 | 0.25 | 2.05 | 1.04 | 3.68 | 1.66 |
| **T1/2 a (min)** | 0.26 | 0.06 | 0.39 | 0.24 | 0.10 | 0.05 | 0.34 | 0.11 |

La concentration sanguine en dichlorhydrate de cinchonine 20 minutes après l'administration, est supérieure à celle du monochlorhydrate de cinchonine. De plus, le temps d'absorption du dichlorhydrate de cinchonine est également supérieur à celui du monochlorhydrate de cinchonine.

Ces résultats démontrent que le dichlorhydrate de cinchonine a des propriétés de biodisponibilité améliorées vis-à-vis du monochlorhydrate de cinchonine.

## Revendications

1. Composition pharmaceutique comprenant, comme principe actif, du dichlorhydrate de cinchonine.

2. Composition pharmaceutique selon la revendication 1 dans laquelle le dichlorhydrate de cinchonine présente une pureté au moins égale à 95 %, de préférence au moins égale à 99 %, et plus préférentiellement encore au moins égale à 99,9 %.

3. Composition pharmaceutique selon la revendication 1, comprenant au moins un autre principe actif compatible avec le dichlorhydrate de cinchonine et éventuellement au moins un autre composé pharmaceutiquement acceptable.

4. Composition pharmaceutique selon la revendication 3, **caractérisée en ce que** les autres principes actifs sont choisis dans le groupe comprenant les agents chimiothérapeutiques, antimitotiques, antipaludiens et inhibiteurs de la résistance multidrogue.

5. Procédé de préparation d'une composition pharmaceutique selon l'une quelconque des revendications 1 ou 2, **caractérisé par le fait que** l'on ajoute de l'acide chlorhydrique à la cinchonine ou au monochlorhydrate de cinchonine afin d'obtenir le dichlorhydrate de cinchonine, que l'on purifie la cinchonine, le monochlorhydrate de cinchonine et/ou le dichlorhydrate de cinchonine et, éventuellement, que l'on soumette le dichlorhydrate de cinchonine ainsi obtenu à une filtration stérilisante et/ou à un séchage par lyophilisation.

6. Procédé selon la revendication 5, **caractérisé par le fait que** l'on purifie la cinchonine, le monochlorhydrate de cinchonine et/ou le dichorhydrate de cinchonine par chromatographie liquide haute performance.

7. Procédé selon la revendication 5, **caractérisé par le fait que** l'on ajoute l'acide chlorhydrique en deux étapes, la cinchonine étant dans une première étape transformée en monochlorhydrate de cinchonine, puis, dans une deuxième étape, le monochlorhydrate de cinchonine étant transformé en dichlorhydrate de cinchonine et, éventuellement, que l'on purifie la cinchonine, le monochlorhydrate de cinchonine et/ou le dichlorhydrate de cinchonine par chromatographie liquide haute performance.

8. Procédé selon la revendication 5, **caractérisé par le fait que** l'on neutralise la cinchonine en une seule étape, et éventuellement que l'on purifie la cinchonine et/ou le dichlorhydrate de cinchonine obtenu par chromatographie liquide haute performance.

9. Utilisation de la composition pharmaceutique telle que définie dans l'une des revendications 1 à 4 pour l'obtention d'un médicament destiné au traitement du phénomène de la résistance multidrogue dans les cancers.

10. Utilisation de la composition pharmaceutique telle que définie dans l'une quelconque des revendications 1 à 4 pour l'obtention d'un médicament destiné au traitement de l'arythmie.

11. Utilisation de la composition pharmaceutique telle que définie dans l'une quelconque des revendications 1 à 4 pour l'obtention d'un médicament antispasmodique.

12. Utilisation de la composition pharmaceutique telle que définie dans la revendication 4 pour l'obtention d'un médicament destiné au traitement du paludisme.

## Patentansprüche

1. Pharmazeutische Zusammensetzung, die als Wirkstoff Cinchonindihydrochlorid enthält.

2. Pharmazeutische Zusammensetzung nach Anspruch 1, in der das Cinchonindihydrochlorid eine Reinheit aufweist, die wenigstens gleich 95 %, vorzugsweise wenigstens gleich 99 % und am besten wenigstens gleich 99,9 % ist.

3. Pharmazeutische Zusammensetzung nach Anspruch 1, die wenigstens einen weiteren Wirkstoff, der mit dem Cinchonindihydrochlorid verträglich ist, und möglicherweise wenigstens eine weitere pharmazeutisch taugliche Verbindung umfaßt.

4. Pharmazeutische Zusammensetzung nach Anspruch 3, **dadurch gekennzeichnet, daß** die weiteren Wirkstoffe aus der Gruppe gewählt sind, welche die chemotherapeutischen und antimitotischen Agenzien, Antisumpffieber-Agenzien und Hemmer von Multiple Drug Resistance (MDR) umfaßt.

5. Verfahren zur Aufbereitung einer pharmazeutischen Zusammensetzung nach einem der Ansprüche 1 oder 2, **dadurch gekennzeichnet, daß** Salzsäure dem Cinchonin oder dem Cinchoninmonohydrochlorid zugesetzt wird, um das Cinchonindihydrochlorid zu erhalten, daß das Cinchonin, das Cinchoninmonohydrochlorid und/oder das Cinchonindihydrochlorid gereinigt werden und daß möglicherweise das so erhaltene Cinchonindihydrochlorid einer sterilisierenden Filterung und/oder einer Trocknung durch Lyophilisation unterzogen wird.

6. Verfahren nach Anspruch 5, **dadurch gekennzeichnet, daß** das Cinchonin, das Cinchoninmonohydrochlorid und/oder das Cinchonindihydrochlorid durch Hochleistungsflüssigchromatographie gereinigt werden.

7. Verfahren nach Anspruch 5, **dadurch gekennzeichnet, daß** Salzsäure in zwei Schritten zugesetzt wird, wobei das Cinchonin in einem ersten Schritt in Cinchoninmonohydrochlorid umgeformt wird, dann in einem zweiten Schritt das Cinchoninmonohydrochlorid in Cinchonindihydrochlorid umgeformt wird, und daß möglicherweise das Cinchonin, das Cinchoninmonohydrochlorid und/oder das Cinchonindihydrochlorid durch Hochleistungsflüssigchromatographie gereinigt werden.

8. Verfahren nach Anspruch 5, **dadurch gekennzeichnet, daß** das Cinchonin in einem einzigen Schritt neutralisiert wird und daß möglicherweise das Cinchonin und/oder das erhaltene Cinchonindihydrochlorid durch Hochleistungsflüssigchromatographie gereinigt werden.

9. Verwendung der pharmazeutischen Zusammensetzung, so wie sie in einem der Ansprüche 1 bis 4 definiert ist, zur Erlangung eines Medikamentes, das zur Behandlung der Erscheinung der Multiple Drug Resistance (MDR) bei Krebskrankheiten bestimmt ist.

10. Verwendung der pharmazeutischen Zusammensetzung, so wie sie in einem der Ansprüche 1 bis 4 definiert ist, zur Erlangung eines Medikamentes, das zur Behandlung der Arrythmie bestimmt ist.

11. Verwendung der pharmazeutischen Zusammensetzung, so wie sie in einem der Ansprüche 1 bis 4 definiert ist, zur Erlangung eines antispasmodischen Medikamentes.

12. Verwendung der pharmazeutischen Zusammensetzung, so wie sie in einem der Ansprüche 1 bis 4 definiert ist, zur Erlangung eines Medikamentes, das zur Behandlung von Sumpffieber bestimmt ist.

## Claims

1. A pharmaceutical composition containing, as active ingredient, cinchonine dihydrochloride.

2. A pharmaceutical composition according to Claim 1 in which the cinchonine dihydrochloride has a purity of at least 95%, preferably at least 99%, in particular at least 99.9%.

3. A pharmaceutical composition according to Claim 1, containing at least one other active ingredient compatible with cinchonine dihydrochloride and optionally at least one other pharmaceutically acceptable compound.

4. A pharmaceutical composition according to Claim 3, wherein the other active ingredients are chosen from the group consisting of chemotherapeutic, antimitotic and antimalarial agents and multiple drug resistance inhibitors.

5. A process for preparing a pharmaceutical composition according to anyone of claims 1 or 2, wherein hydrochloric acid is added to cinchonine or to cinchonine monohydrochloride in order to obtain cinchonine dihydrochloride, the cinchonine, cinchonine monohydrochloride and/or cinchonine dihydrochloride are purified and, optionally, the cinchonine dihydrochloride thus obtained is subjected to sterilising filtration and/or drying by freeze-drying.

6. A process according to Claim 5, wherein the cinchonine, cinchonine monohydrochloride and/or cinchonine dihydrochloride are purified by high performance liquid chromatography.

7. A process according to Claim 5, wherein the hydrochloric acid is added in two stages, the cinchonine being transformed into cinchonine monohydrochloride in a first stage, then, in a second stage, the cinchonine monohydrochloride being transformed into cinchonine dihydrochloride and, optionally, the cinchonine, cinchonine monohydrochloride and/or cinchonine dihydrochloride being purified by high performance liquid chromatography.

8. A process according to Claim 5, wherein the cinchonine is neutralised in a single stage and, optionally, the cinchonine and/or cinchonine dihydrochloride obtained are purified by high performance liquid chromatography.

9. Use of the pharmaceutical composition as defined in one of claims 1 to 4 for obtaining a medicament for the treatment of the multiple drug resistance phenomenon in cancers.

10. Use of the pharmaceutical composition as defined in one of claims 1 to 4 for obtaining a medicament for treating arrhythmia.

11. Use of the pharmaceutical composition as defined in one of claims 1 to 4 for obtaining an antispasmodic substance.

12. Use of the pharmaceutical composition as defined in claim 4 for obtaining a medicament in the treatment of malaria.
